# EUROPÄISCHE PATENTSCHRIFT

(11) **EP 0 820 988 B1**
(45) Veröffentlichungstag und Bekanntmachung des Hinweises auf die Patenterteilung: **01.07.2009**
(21) Anmeldenummer: 97112196.7
(22) Anmeldetag: 17.07.1997
(51) Int. Cl.: C07D 233/52, A61K 31/415

(54) **Imino-Derivate als Inhibitoren der Knochenresorption und Vitronectinrezeptor-Antagonisten**
Imino derivatives as bone resorption inhibitors and vitronectin receptor antagonists
Dérivés imino comme inhibiteurs de la résorption osseuse et comme antagonistes du récepteur de la vitronectine

(30) Priorität: 24.07.1996 DE 19629817
(43) Veröffentlichungstag der Anmeldung: 28.01.1998
(73) Patentinhaber: HOECHST AKTIENGESELLSCHAFT, 65929 Frankfurt am Main (DE); Genentech, Inc., South San Francisco CA 94080 (US)
(72) Erfinder: Wehner, Volkmar, Dr., 97657 Sandberg (DE); Knolle, Jochen, Dr., 65830 Kriftel (DE); Stilz, Hans Ulrich, Dr., 65929 Frankfurt (DE); Gourvest, Jean-Francois, Dr., 77410 Claye Souilly (FR); Carniato, Denis, Dr., 91460 Marcoussis (FR); Gadek, Thomas Richard, Dr., Oakland, CA 94611 (US); McDowell, Robert, Dr., San Francisco, CA 94114 (US); Pitti, Robert Maurice, El Cerrito, CA 94530 (US); Bodary, Sarah Catherine, Dr., San Bruno, CA 94066 (US)
(74) Vertreter: Rousseau, Pierrick Edouard

(56) Entgegenhaltungen:
- EP-A- 0 528 586
- EP-A- 0 528 587
- EP-A- 0 566 919
- WO-A-93/18057
- WO-A-94/08577
- WO-A-94/12181
- WO-A-95/14008
- WO-A-95/32710
- WO-A-96/00574
- WO-A-96/00730
- DE-A- 2 745 596
- N. DESIDERI ET AL.: ARCH. PHARM., Bd. 325, Nr. 12, 1992, Seiten 773-7, XP000864378

## Beschreibung

Gegenstand der vorliegenden Erfindung sind Verbindungen der Formel I sowie deren physiologisch verträgliche Salze und solche Verbindungen enthaltende pharmazeutische Zubereitungen, deren Herstellung und Verwendung als Arzneimittel, insbesondere als Inhibitoren der Knochenresorption durch Osteoclasten, als Inhibitoren von Tumorwachstum und Tumormetastasierung, als Entzündungshemmer, zur Behandlung oder Prophylaxe von cardiovaskulären Erkrankungen wie Arteriosklerose oder Restenose, zur Behandlung oder Prophylaxe von Nephropathien und Retinopathien, wie z.B. diabetischer Retinopathie, sowie als Vitronectinrezeptor-Antagonisten zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen. Die Erfindung betrifft ferner die Verwendung der Verbindungen der Formel I sowie deren physiologisch verträglichen Salze und solche Verbindungen enthaltende pharmazeutische Zubereitungen als Arzneimittel zur Linderung oder Heilung von Krankheiten, die zumindest teilweise durch ein unerwünschtes Maß an Knochenresorption, Angiogenese, oder Proliferation von Zellen der glatten Gefäßmuskulatur bedingt sind.

Die menschlichen Knochen unterliegen einem fortwährenden dynamischen Umbauprozeß, der Knochenresorption und Knochenaufbau beinhaltet. Diese Prozesse werden von dafür spezialisierten Zelltypen gesteuert. Knochenaufbau beruht auf der Ablagerung von Knochenmatrix durch Osteoblasten, Knochenresorption beruht auf dem Abbau von Knochenmatrix durch Osteoclasten. Die Mehrzahl der Knochenerkrankungen beruhen auf einem gestörten Gleichgewicht zwischen Knochenbildung und Knochenresorption. Osteoporose ist charakterisiert durch einen Verlust an Knochenmatrix. Aktivierte Osteoclasten sind vielkernige Zellen mit einem Durchmesser bis zu 400 µm, die Knochenmatrix abtragen. Aktivierte Osteoclasten lagern sich an die Oberfläche der Knochenmatrix an und sezernieren proteolytische Enzyme und Säuren in die sogenannte "sealing zone", dem Bereich zwischen ihrer Zellmembran und der Knochenmatrix. Die saure Umgebung und die Proteasen bewirken den Abbau des Knochens.

Die erfindungsgemäßen Verbindungen der Formel I inhibieren die Knochenresorption durch Osteoclasten. Knochenkrankheiten gegen die die erfindungsgemäßen Verbindungen eingesetzt werden können sind vor allem Osteoporose, Hypercalcämie, Osteopenie, z.B. hervorgerufen durch Metastasen, Zahnerkrankungen, Hyperparathyroidismus, periarticulare Erosionen bei rheumathoider Arthritis und Paget Krankheit.
Ferner können die Verbindungen der Formel I zur Linderung, Vermeidung oder Therapie von Knochenerkrankungen, die durch eine Glucocortikoid-, Steroid- oder Corticosteroid-Therapie oder durch einen Mangel an Sexualhormon(en) hervorgerufen werden, eingesetzt werden. Alle diese Erkrankungen sind durch Knochenverlust gekennzeichnet, der auf dem Ungleichgewicht zwischen Knochenaufbau und Knochenabbau beruht.

Studien haben gezeigt, daß die Anlagerung von Osteoclasten an den Knochen durch Integrin-Rezeptoren auf der Zelloberfläche von Osteoclasten gesteuert wird.

Integrine sind eine Superfamilie von Rezeptoren zu denen unter anderen der Fibrinogenrezeptor α_{IIb}β₃ auf den Blutplättchen und der Vitronectinrezeptor αᵥβ₃ gehören. Der Vitronectinrezeptor αᵥβ₃ ist ein membranständiges Glycoprotein, das auf der Zelloberfläche einer Reihe von Zellen wie Endothelzellen, Zellen der glatten Gefäßmuskulatur, Osteoclasten und Tumorzellen exprimiert wird. Der Vitronectinrezeptor α_{V}β₃, der auf der Osteoclastenmembran exprimiert wird steuert den Prozeß der Anlagerung an den Knochen und der Knochenresorption und trägt somit zur Osteoporose bei.

α_{V}β₃ bindet hierbei an Knochenmatrixproteine wie Osteopontin, Knochensialoprotein und Thrombospontin, die das Tripeptidmotif Arg-Gly-Asp (oder Horton und Mitarbeiter beschreiben RGD-Peptide und einen anti-Vitronectinrezeptor Antikörper (23C6), die den Zahnabbau durch Osteoclasten und das Wandern von Osteoclasten inhibieren (Horton et al., Exp. Cell. Res. 1991, 195, 368). Sato et al. beschreiben in J. Cell Biol. 1990, 111, 1713 Echistatin, ein RGD-Peptid aus Schlangengift, als potenten Inhibitor der Knochenresorption in einer Gewebekultur und als Hemmstoff der Osteoclasten-Anheftung an den Knochen. Fischer et al. (Endocrinology, 1993, 132, 1411) konnten an der Ratte zeigen, daß Echistatin die Knochenresorption auch in vivo hemmt.

Der Vitronectinrezeptor α_{V}β₃ auf humanen Zellen der glatten Gefäßmuskulatur der Aorta stimuliert die Wanderung dieser Zellen in das Neointima, was schließlich zu Arteriosklerose und Restenose nach Angioplastie führt (Brown et al., Cardiovascular Res. 1994, 28, 1815).

Die Verbindungen der Formel I können ferner als Träger von Wirkstoffen dienen, um den Wirkstoff gezielt an den Wirkort zu transportieren (= Drug Targeting, siehe z.B. Targeted Drug Delivery, R.C. Juliano, Handbook of Experimental Pharmacology Vol. 100, Ed. Born, G.V.R. et al., Springer Vorlag). Bei den Wirkstoffen handelt es sich um solche, die zur Behandlung der obengenannten Krankheiten verwendet werden können.

Brooks et al. (Cell 1994, 79, 1157) zeigen, daß Antikörper gegen α_{V}β₃ oder α_{V}β₃-Antagonisten eine Schrumpfung von Tumoren bewirken können, indem sie die Apoptose von Blutgefäßzellen während der Angiogenese induzieren. Chersh et al. (Science 1995, 270, 1500) beschreiben anti α_{V}β₃-Antikörper oder α_{V}β₃-Antagonisten, die bFGF induzierte Angiogeneseprozesse im Rattenauge inhibieren, was therapeutisch bei der Behandlung von Retinopathien nützlich sein könnte.

Die Patentanmeldung DE 27 45 596 betrifft neue Guanylhydrazone mit antimithotischen, IMAO- und plättchen-antiaggregierenden Wirkung.

In der Patentanmeldung WO 94/12181 werden substituierte aromatische oder nichtaromatische Ringsysteme, in WO 94/08577 substituierte Heterocyclen als Fibrinogenrezeptor-Antagonisten und Inhibitoren der Plättchenaggregation beschrieben. Aus EP-A-518 586 und EP-A-528 587 sind Aminoalkyl- oder Heterocyclyl-substituierte Phenylalanin-Derivate, aus WO 95/32710 Arylderivate als Hemmstoffe der Knochenresorption durch Osteoclasten bekannt. Die WO 96/00574 beschreibt Benzodiazepine, die WO 96/00730 Fibrinogenrezeptorantagonisten-Template, insbesondere Benzodiazepine, die an einen Stickstoff tragenden 5-Ring geknüpft sind, als Vitronectinrezeptor-Antagonisten.

Gegenstand der vorliegenden Erfindung sind Verbindungen der Formel I,

R¹-Y-A-B-D-E-F-G I,

worin bedeuten:
- A: -NH-N = CH-;
- B: (C₁-C₆)-Alkandiyl;
- D: -O-;
- E: Phenylen;
- F: -CO-NH-;
- G: 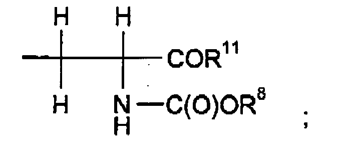
- Y: eine direkte Bindung;
- R¹: H₂N-C(=NH)-, 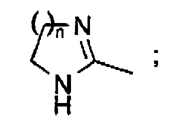
- R⁸: H, (C₁-C₆)-Alkyl, (C₅-C₆)-Cycloalkyl, (C₅-C₆)-Cycloalkyl-(C₁-C₂)-alkandiyl, (C₅-C₆)-Aryl, (C₅-C₆)-Aryl-(C₁-C₂)-alkandiyl;
- R¹¹: OH, (C₁-C₆)-Alkoxy, Phenoxy, Benzyloxy, (C₁-C₄)-Alkylcarbonyloxy-(C₁-C₄)-alkandiyloxy, NH₂, Mono- oder Di-(C₁-C₆)-alkyl-amino;
- n: 1 oder 2;
in allen ihren stereoisomeren Formen und Gemische davon in allen Verhältnissen, und ihre physiologisch verträglichen Salze.

Die in den Substituenten auftretenden Alkylreste können geradkettig oder verzweigt, gesättigt oder einfach oder mehrfach ungesättigt sein. Entsprechendes gilt für davon abgeleitete Reste, wie z.B. Alkoxy. Cycloalkylreste können mono-, bi- oder tricyclisch sein.

Monocyclische Cycloalkylreste sind insbesondere Cyclopropyl, Cyclobutyl, Cyclopentyl, Cyclohexyl, Cycloheptyl und Cyclooctyl, die aber auch durch beispielsweise (C₁-C₄)-Alkyl substituiert sein können. Als Beispiele für substituierte Cycloalkylreste seien 4-Methylcyclohexyl und 2,3-Dimethylcyclopentyl genannt.

Bicyclische und tricyclische Cycloalkylreste können unsubstituiert sein oder in beliebigen geeigneten Positionen durch eine oder mehrere Oxogruppen und/oder eine oder mehrere gleiche oder verschiedene (C₁-C₄)-Alkylgruppen, z.B. Methyl- oder Isopropylgruppen, bevorzugt Methylgruppen, substituiert sein. Die freie Bindung des bi- oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo- oder einer endo-Position.

Beispiele für 6-gliedrige aromatische Ringsysteme sind Phenyl, Pyridyl, Pyridazinyl, Pyrimidinyl, Pyrazinyl, 1,3,6-Triazinyl, 1,2,4-Triazinyl, 1,2,3-triazinyl, Tetrazinyl.

Beispiele für Grundkörper bicyclischer Ringsysteme sind das Norbornan (= Bicyclc[2.2.1]heptan), das Bicyclo[2.2.2]octan und das Bicyclo[3.2.1]octan. Ein Beispiel für ein mit einer Oxogruppe substituiertes System ist der Campher (= 1,7,7-trimethyl-2-oxobicyclo[2.2.1]heptan).

Beispiele für Grundkörper tricyclischer Systeme sind das Twistan (= Tricyclo[4.4.0.0^{3,8}]decan, das Adamantan (= Tricyclo[3.3.1.1^{3,7}]decan), das Noradamantan (= Tricyclo[3.3.1.0^{3,7}]nonan), das Tricyclo[2.2.1.0^{2,6}]heptan, das Tricyclo[5.3.2.0^{4,9}]dodecan, das Tricyclo[5.4.0.0^{2,9}]undecan oder das Tricyclo[5.5.1.0^{3,11}]tridecan.

Aryl ist beispielsweise Phenyl, Naphthyl, Biphenylyl, Anthryl oder Fluorenyl, wobei 1-Naphthyl, 2-Naphthyl und insbesondere Phenyl bevorzugt sind. Arylreste, insbesondere Phenylreste, können ein- oder mehrfach, bevorzugt ein-, zwei oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, wie Fluor, Chlor und Brom, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyl, Benzyloxy, Tetrazolyl, (R¹⁷O)₂P(O)- und (R¹⁷O)₂P(O)-O-, wobei R¹⁷ für H, (C₁-C₁₀)-Alkyl, (C₆-C₁₄)-Aryl oder (C₆-C₁₄)-Aryl-(C₁-C₈)-alkyl steht, substituiert sein.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Bevorzugt sind in zweifach substituierten Phenylresten die beiden Substituenten in der 3- und der 4-Position, bezogen auf die Verknüpfungsstelle, angeordnet.

Arylgruppen können ferner mono- oder polycyclische aromatische Ringsysteme darstellen, worin 1 bis 5 C-Atome durch 1 bis 5 Heteroatome ersetzt sein können, wie z.B. 2-Pyridyl, 3-Pyridyl, 4-Pyridyl, Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benz-anelliertes, cyclopenta-, cyclohexa- oder cyclohepta-anelliertes Derivat dieser Reste.

Diese Heterocyclen können mit den gleichen Substituenten wie die vorstehend genannten carbocyclischen Arylsysteme substituiert sein.

In der Reihe dieser Arylgruppen sind bevorzugt mono- oder bicyclische aromatische Ringsysteme mit 1 - 3 Heteroatome aus der Reihe N, O, S, die mit 1 - 3 Substituenten aus der Reihe (C₁-C₆)-Alkyl, (C₁-C₆)-Alkoxy, Fluor, Cl, NO₂, NH₂, Trifluormethyl, OH, (C₁-C₄)-Alkoxycarbonyl, Phenyl, Phenoxy, Benzyloxy oder Benzyl substituiert sein können.

Besonders bevorzugt sind hierbei mono- oder bicyclische aromatische 5 - 10 Ringsysteme mit 1 - 3 Heteroatomen aus der Reihe N, O, S, die mit 1 - 2 Substituenten aus der Reihe (C₁-C₄)-Alkyl, (C₁-C₄)-Alkoxy, Phenyl, Phenoxy, Benzyl oder Benzyloxy substituiert sein können.

Bevorzugt sind auch Verbindungen der Formel I, die einen lipophilen Rest R⁴, R⁵, R⁶ oder R⁷ wie z.B. Benzyloxycarbonylamino, Cyclohexylmethylcarbonylamino etc. tragen.

L- oder D-Aminosäuren können natürliche oder unnatürliche Aminosäuren sein. Bevorzugt sind α-Aminosäuren. Beispielsweise seien genannt (vgl. Houben-Weyl, Methoden der organischen Chemie, Band XV/1 und 2, Georg Thieme Verlag, Stuttgart, 1974):
Aad, Abu, γAbu, ABz, 2ABz, eAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, tert. Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure;
ferner:
Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]-heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 2-Azatricyclo[4.3.0.1^{6,9}]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetahydrothiazol-4-carbonsäure; Isoxazolidin-3-carbonsäure; Pyrazolidin-3-carbonsäure, Hydroxypyrrolidin-2-carbonsäure, die alle gegebenenfalls substituiert sein können (siehe folgende Formeln):

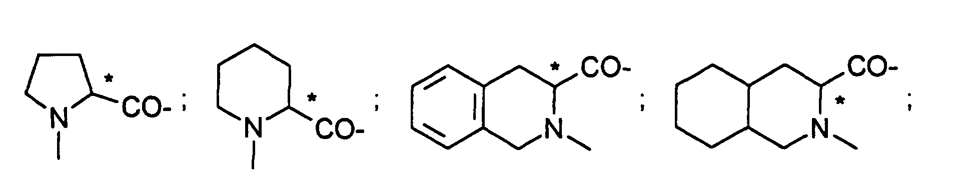

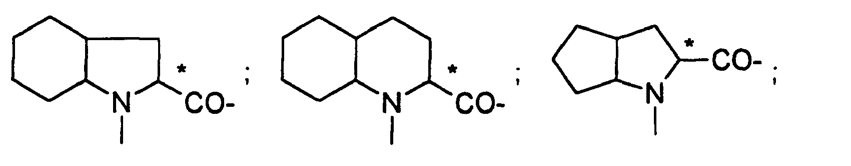

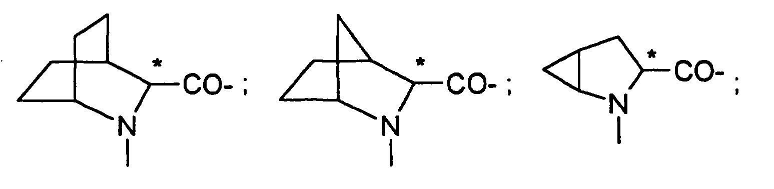

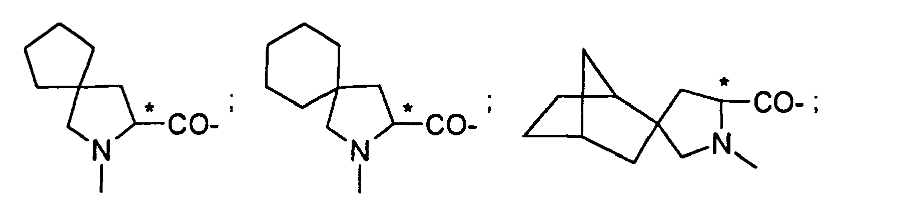

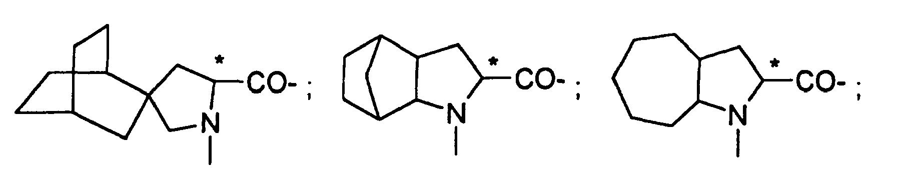

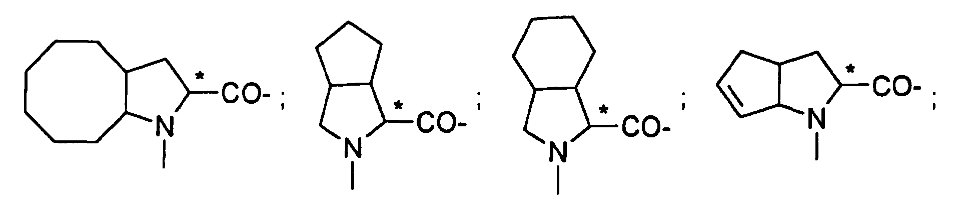

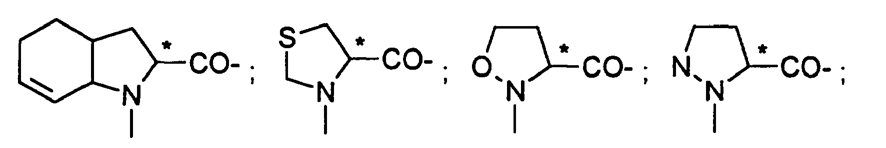

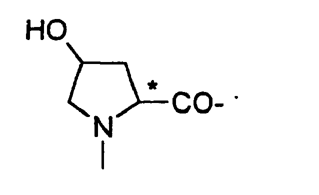

Die oben genannten Resten zugrundeliegenden Heterocyclen sind beispielsweise bekannt aus US-A-4,344,949; US-A 4,374,847;
US-A 4,350,704; EP-A 29,488; EP-A 31,741; EP-A 46,953; EP-A 49,605;
EP-A 49,658; EP-A 50, 800; EP-A 51,020; EP-A 52,870; EP-A 79,022;
EP-A 84,164; EP-A 89,637; EP-A 90,341; EP-A 90,362; EP-A 105,102;
EP-A 109,020; EP-A 111,873; EP-A 271,865 und EP-A 344,682.

Ferner können die Aminosäuren auch als Ester bzw. Amide vorliegen, wie z.B. Methylester, Ethylester, Isopropylester, Isobutylester, tert.-Butylester, Benzylester, Ethylamid, Semicarbazid oder ω-Amino-(C₂-C₈)-alkylamid.

Funktionelle Gruppen der Aminosäuren können geschützt vorliegen. Geeignete Schutzgruppen wie z.B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert.-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze. Solche Salze werden beispielsweise von Verbindungen der Formel I, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin, Ethanolamin oder Tris-(2-hydroxy-ethyl)-amin. Verbindungen der Formel I, welche basische Gruppen, z.B. eine Aminogruppe, eine Amidinogruppe oder eine Guanidinogruppe enthalten, bilden mit anorganischen Säuren, wie z.B. Salzsäure, Schwefelsäure oder Phosphorsäure, und mit organischen Carbon- oder Sulfonsäuren, wie z.B. Essigsäure, Citronensäure, Benzoesäure, Maleinsäure, Fumarsäure, Weinsäure, Methansulfonsäure oder p-Toluolsulfonsäure, Salze.

Die erfindungsgemäßen Verbindungen der Formel I können optisch aktive Kohlenstoffatome, die unabhängig voneinander R- oder S-Konfiguration haben können, enthalten und somit in Form reiner Enantiomerer oder reiner Diastereomerer oder in Form von Enantiomerengemischen oder Diastereomerengemischen vorliegen. Sowohl reine Enantiomere und Enantiomerengemische als auch Diastereomere und Diastereomerengemische sind Gegenstand der vorliegenden Erfindung. Die Erfindung umfaßt Gemische von zwei und von mehr als zwei Stereoisomeren der Formel I and alle Verhältnisse der Stereoisomeren in den Gemischen.

Die erfindungsgemäßen Verbindungen der Formel I können, da mindestens einer der Reste A, D oder F unabhängig voneinander -NR²-N=CR²-, -N=CR²- oder -R²C=N- ist, und falls einer oder mehrere Reste in der Formel I für -CR²=CR³- steht, als E/Z-Isomerengemische vorliegen. Gegenstand der vorliegenden Erfindung sind sowohl reine E- bzw. Z-Isomere als auch Gemische von E/Z-Isomeren in allen Verhältnissen. Diastereomere einschließlich E/Z-Isomere können durch Chromatographie in die Einzelisomeren aufgetrennt werden. Racemate können entweder durch Chromatographie an chiralen Phasen oder durch Racematspaltung in die beiden Enantiomere aufgetrennt werden.

Die erfindungsgemäßen Verbindungen der Formel I können darüber hinaus bewegliche Wasserstoffatome enthalten, also in verschiedenen tautomeren Formen vorliegen. Auch diese Tautomere sind Gegenstand der vorliegenden Erfindung.

Verbindungen der Formel I können generell, beispielsweise im Zuge einer konvergenten Synthese, durch Verknüpfung zweier oder mehrerer Fragmente, die sich retrosynthetisch aus der Formel I ableiten lassen, hergestellt werden. Bei der Herstellung der Verbindungen der Formel I kann es generell im Laufe der Synthese nötig sein, funktionelle Gruppen, die im jeweiligen Syntheseschritt zu unerwünschten Reaktionen oder Nebenreaktionen führen könnten, durch eine dem Syntheseproblem angepaßte Schutzgruppenstrategie temporär zu blockieren, was dem Fachmann bekannt ist. Die Methode der Fragmentverknüpfung ist nicht auf die nachfolgenden Beispiele beschränkt, sondern allgemein für Synthesen der Verbindungen der Formel I anwendbar.

Beispielsweise können Verbindungen der Formel I des Typs,

R¹-Y-A-B-C-E-C(O)NR²-G,

in dem F in der Formel I gleich -C(O)NR²- ist, worin R² bedeutet H, durch Kondensation einer Verbindung der Formel II,

R¹-Y-A-B-D-E-M II,

wobei M für Hydroxycarbonyl, (C₁-C₆)-Alkoxycarbonyl, aktivierte Carbonsäurederivate wie Säurechloride, Aktivester oder gemischte Anhydride steht, mit HNR²-G hergestellt werden.

Zur Kondensation zweier Fragmente unter Bildung einer Amidbindung verwendet man vorteilhafterweise die an sich bekannten Kupplungsmethoden der Peptidchemie (siehe z.B. Houben-Weyl, Methoden der Organischen Chemie, Band 15/1 und 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert.-Butylester eingesetzt werden. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen In geeigneter Weise abgespalten. Beispielsweise können NO₂-Gruppen (Guanidinoschutz), Benzyloxycarbonylgruppen und Benzylester abhydriert werden. Die Schutzgruppen vom tert.-Butyltyp werden sauer abgespalten, während der 9-Fluorenylmethyloxycarbonylrest durch sekundäre Amine entfernt wird.

Verbindungen der Formel I, in denen R¹-Y-A- für 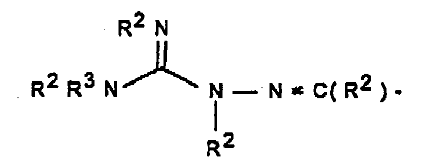 oder cyclische Guanylhydrazone des Typs 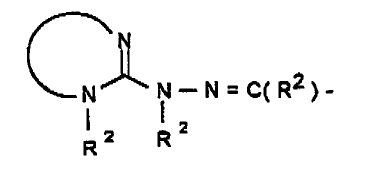 steht, worin R² und R³ bedeuten H, werden beispielsweise durch Kondensation von 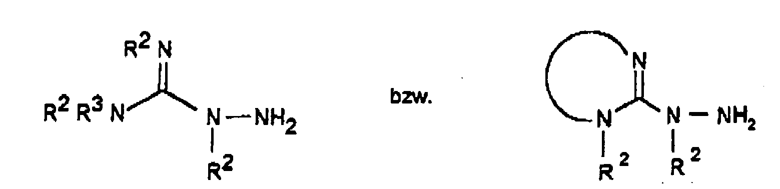 mit Ketonen oder Aldehyden des Typs O=C(R²)- oder entsprechenden Acetalen oder Ketalen nach gängigen Literaturverfahren, beispielsweise analog N. Desideri et al., Arch. Pharm. 325 (1992) 773-777, A. Alves et al., Eur. J. Med. Chem. Chim. Ther. 21 (1986) 297-304, D. Heber et al., Pharmazie 50 (1995) 663-667, T.P. Wunz et al., J. Med. Chem. 30 (1987) 1313-1321, K-H. Buchheit et al., J. Med. Chem. 38 (1995), 2331-2338, oder wie in Beispiel 1 beschrieben (Kondensation unter Salzsäure-Katalyse in Eisessig) hergestellt.

Obige Guanylhydrazone können gegebenenfalls als E/Z-Isomerengemische anfallen, die nach gängigen Chromatographieverfahren getrennt werden können.

Verbindungen der Formel I, in denen F für -C(O)NR²- steht, worin R² bedeutet H, können z.B. durch Umsetzung von

R¹-Y-A-B-D-E-C(O)Q

(Q ist eine leicht nukleophil substituierbare Abgangsgruppe, wie z.B. OH, Cl, OCH₃ etc.) mit HR²N-G bzw. HO-G nach Literaturverfahren erhalten werden.

Die Verbindungen der Formel I and ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Salzes davon, neben üblichen pharmazeutisch einwandfreien Träger- und Zusatzstoffen enthalten. Die Zubereitungen enthalten normalerweise etwa 0,5 bis 90 Gew.-% der therapeutisch wirksamen Verbindung.

Die Arzneimittel können oral, z.B. in Form von Pillen, Tabletten, Lacktabletten, Dragees, Granulaten, Hart- und Weichgelatinekapseln, Lösungen, Sirupen, Emulsionen, Suspensionen oder Aerosolmischungen verabreicht werden. Die Verabreichung kann aber auch rektal, z.B. in Form von Suppositorien, oder parenteral, z.B. in Form von injektions- oder Infusionslösungen, Mikrokapseln oder Rods, perkutan, z.B. in Form von Salben oder Tinkturen, oder nasal, z.B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder deren physiologisch verträglichen Salze enthalten; ferner neben mindestens einer Verbindung der Formel I noch einen oder mehrere andere therapeutisch wirksame Stoffe.

Die Dosis kann innerhalb weiter Grenzen variieren und ist in jedem einzelnen Fall den individuellen Gegebenheiten anzupassen. Bei oraler Verabreichung beträgt die Tagesdosis im allgemeinen 0,01 bis 50 mg/kg, vorzugsweise 0,1 bis 5 mg/kg, insbesondere 0,3 bis 0,5 mg/kg Körpergewicht zur Erzielung wirksamer Ergebnisse. Bei intravenöser Applikation beträgt die Tagesdosis im allgemeinen etwa 0,01 bis 100 mg/kg, vorzugsweise 0,05 bis 10 mg/kg Körpergewicht. Die Tagesdosis kann, insbesondere bei der Applikation größerer Mengen, in mehrere, z.B. 2, 3 oder 4, Teilverabreichungen aufgeteilt werden. Gegebenenfalls kann es, je nach individuellem Verhalten, erforderlich werden, von der angegebenen Tagesdosis nach oben oder nach unten abzuweichen.

Die Hemmung der Knochenresorption durch die erfindungsgemäßen Verbindungen kann beispielsweise mit Hilfe eines Osteoclasten-Resorptions-Tests ("PIT ASSAY") beispielsweise analog WO 95/32710 bestimmt werden. Testmethoden, nach denen die antagonistische Wirkung der erfindungsgemäßen Verbindungen auf den Vitronectinrezeptor αᵥβ₃ bestimmt werden können, sind nachfolgend beschrieben.

### Testmethode 1:

Inhibierung der Bindung von humanem Vitronectin (Vn) an humanen Vitronectinrezeptor (VnR) αᵥβ₃ (ELISA-Test)
1. Reinigung von humanem Vitronectin
   Humanes Vitronectin wird aus menschlichem Plasma isoliert und durch Affinitätschromatographie nach der Methode von Yatohyo et al., Cell Structure and Function, 1988, 23, 281-292 gereinigt.
2. Reinigung von humanem Vitronectinrezeptor (αᵥβ₃)
   Humaner Vitronectinrezeptor wird aus der menschlichen Plazenta nach der Methode von Pytela et al., Methods Enzymol. 1987, 144, 475 gewonnen. Humaner Vitronectinrezeptor αᵥβ₃ kann auch aus einigen Zellinien (z.B. aus 293 Zellen, einer humanen embryonalen Nierenzellinie), die mit DNA Sequenzen für beide Untereinheiten αᵥ und β₃ des Vitronectinrezeptors cotransfiziert sind, gewonnen werden. Die Untereinheiten werden mit Octylglycosid extrahiert und anschließend über Concanavalin A, Heparin-Sepharose und S-300 chromatographiert.
3. Monoklonale Antikörper
   Murine monoklonale Antikörper, spezifisch für die β₃ Untereinheit des Vitronectinrezeptors, werden nach der Methode von Newman et al., Blood, 1985, 227-232, oder nach einem ähnlichen Verfahren hergestellt. Das Kaninchen Fab 2 anti-Maus Fc Konjugat an Meerrettich-Peroxidase (anti-Maus Fc HRP) wurde von Pel Freeze (Katalog Nr. 715 305-1) bezogen.
4. ELISA Test
   Nunc Maxisorp 96 well Mikrotiter Platten werden mit einer Lösung von humanem Vitronectin (0,002 mg/ml, 0,05 ml/well) in PBS (Phosphat-gepufferte Kochsalzlösung) über Nacht bei 4°C belegt. Die Platten werden zweimal mit PBS/0,05 % Tween 20 gewaschen und durch Inkubieren (60 min) mit Rinderserum-Albumin (BSA, 0,5 %, RIA Güteklasse oder besser) in Tris-HCl (50 mM), NaCl (100 mM), MgCl₂ (1 mM), CaCl₂ (1 mM), MnCl₂ (1 mM), pH 7, geblockt. Man stellt Lösungen von bekannten Inhibitoren und von den Testsubstanzen in Konzentration von 2x10⁻¹² - 2x10⁻⁶ Mol/l in Assay-Puffer [BSA (0,5 %, RIA-Güteklasse oder besser) in Tris-HCl (50 mM/I), NaCl (100 mM), MgCl₂ (1 mM), CaCl₂ (1 mM), MnCl₂ (1 mM), pH 7] her. Die geblockten Platten werden entleert, und jeweils 0,025 ml dieser Lösung, die eine definierte Konzentration (2x10⁻¹² bis 2x10⁻⁶) entweder an einem bekannten Inhibitor oder an einer Testsubstanz enthält, in jedes well gegeben. 0,025 ml einer Lösung des Vitronectinrezeptors im Testpuffer (0,03 mg/ml) werden in jedes well der Platte pipettiert und die Platte wird auf einem Schüttler 60-180 min bei Raumtemperatur inkubiert. In der Zwischenzeit wird eine Lösung (6 ml/Platte) eines für die β₃-Untereinheit des Vitronectinrezeptors spezifischen murinen monoklonalen Antikörpers im Assay Puffer (0,0015 mg/ml) hergestellt. Zu dieser Lösung gibt man einen zweiten Kaninchen-Antikörper (0,001 ml Stammlösung/6 ml der murinen monoklonalen anti-β₃-Antikörper-Lösung), der ein anti-Maus Fc HRP Antikörper-Konjugat darstellt, und dieses Gemisch aus murinem anti-β₃ Antikörper und Kaninchen anti-Maus Fc HRP Antikörper Konjugat läßt man während der Zeit der Rezeptor-Inhibitor Inkubation inkubieren.
   Die Testplatten werden 4 mal mit PBS-Lösung, die 0,05 % Tween-20 enthält, gewaschen und man pipettiert jeweils 0,05 ml/well der Antikörpermischung in jedes well der Platte und inkubiert 60-180 min. Die Platte wird 4 mal mit PBS/0,05 % Tween-20 gewaschen und anschließend mit 0,05 ml/well einer PBS-Lösung, die 0,67 mg/ml o-Phenylendiamin und 0,012 % H₂O₂ enthält, entwickelt. Alternativ hierzu kann o-Phenylendiamin in einem Puffer (pH 5) eingesetzt werden, der Na₃PO₄ (50 mM) und Zitronensäure (0,22 mM) enthält.
   Die Farbentwicklung wird mit 1 N H₂SO₄ (0,05 ml/well) gestoppt. Die Absorption jedes well wird bei 492-405 nm gemessen und die Daten werden nach Standardmethoden ausgewertet.

### Testmethode 2:

Inhibierung der Bindung von Kistrin an humanen Vitronectinrezeptor (VnR) αᵥβ₃ (ELISA-Test)
(Testmethode 2 wird bei der Auflistung der Testergebnisse mit Kistrin/VnR abgekürzt)
1. Reinigung von Kistrin
   Kistrin wird nach den Methoden von Dennis et al., wie in Proc. Natl. Acad. Sci. USA 1989, 87, 2471-2475 und PROTEINS: Structure, Function and Genetics 1993, 15, 312-321, beschrieben, gereinigt.
2. Reinigung von humanem Vitronectinrezeptor (αᵥβ₃) siehe Testmethode 1
3. Monoklonale Antikörper siehe Testmethode 1
4. ELISA-Test
   Die Fähigkeit von Substanzen die Bindung von Kistrin an den Vitronectinrezeptor zu inhibieren, kann mit einem ELISA-Test ermittelt werden. Zu diesem Zweck werden Nunc 96 well Mikrotiterplatten mit einer Lösung von Kistrin (0,002 mg/ml) nach der Methode von Dennis et al., wie in PROTEINS: Structure, Function and Genetics 1993, 15, 312-321, beschrieben, belegt. Die weitere experimentelle Durchführung des ELISA-Test erfolgt wie bei Testmethode 1, Punkt 4, beschrieben.

### Testmethode 3:

Inhibierung der Bindung von mit αᵥβ₃ transfizierten 293 Zellen an humanes Vitronectin

### Zelltest

293 Zellen, eine humane embryonale Nierenzellinie, die mit DNA-Sequenzen für die αᵥ und β₃ Untereinheiten des Vitronectinrezeptors αᵥβ₃ cotransfiziert sind, werden nach der FACS Methode unter dem Gesichtspunkt einer hohen Expressionsrate (> 500000 αᵥβ₃ Rezeptoren/Zelle) selektiert. Die ausgewählten Zellen werden kultiviert und erneut mittels FACS aussortiert, um eine stabile Zellinie (15 D) mit Expressionsraten > 1000000 Kopien an αᵥβ₃ pro Zelle zu erhalten.

Eine Limbro 96well Gewebekulturplatte mit flachem Boden wird mit humanem Vitronectin (0,01 mg/ml, 0,05 ml/well) in Phosphat-gepufferter Kochsalzlösung (PBS) über Nacht bei 4°C belegt und anschließend mit 0,5 %igem BSA geblockt. Man stellt Lösungen der Testsubstanzen von 10⁻¹⁰ - 2 x 10⁻³ Mol/l in glucosehaltigem DMEM Medium her und gibt jeweils 0,05 ml/well der Lösung auf die Platte. Die Zellen, die hohe Level an αᵥβ₃ exprimieren (z.B. 15 D) werden in glucosehaltigem DMEM Medium suspendiert und die Suspension wird auf einem Gehalt von 25000 Zellen/0,05 ml Medium eingestellt. 0,05 ml dieser Zellsuspension werden in jedes well gegeben und die Platte bei 37°C 90 min inkubiert. Die Platte wird 3 x mit warmen PBS gewaschen, um nichtgebundene Zellen zu entfernen. Die gebundenen Zellen werden in Zitratpuffer (25 mMol, pH 5,0), der 0,25 % Triton X-100 enthält, lysiert. Anschließend wird das Hexoseamidase-Substrat p-Nitrophenyl-N-acetyl-β-D-glucosaminid zugegeben und die Platte 90 min bei 37°C inkubiert. Die Reaktion wird mit einem Glycin (50 mMol)/EDTA (5 mMol)-Puffer (pH 10,4) gestoppt und die Absorption von jedem well bei 405-650 nm gemessen. Die Daten werden nach Standardverfahren ausgewertet.

Es wurden folgende Testergebnisse erhalten:
Kistrin/VnR
IC₅₀ (µM)
Verbindung des Beispiels 1 0,03

### Beispiele

Die Produkte wurden über Massenspektren und/oder NMR-Spektren identifiziert.

### Beispiel 1

4-[2-(N-(Imidazolin-2-yl)hydrazonoethyloxy)]benzoyl-(2S)-2-benzyloxycarbonylamino-β-alanin-Hydrobromid

Die Synthese erfolgte nach folgender Reaktionssequenz:

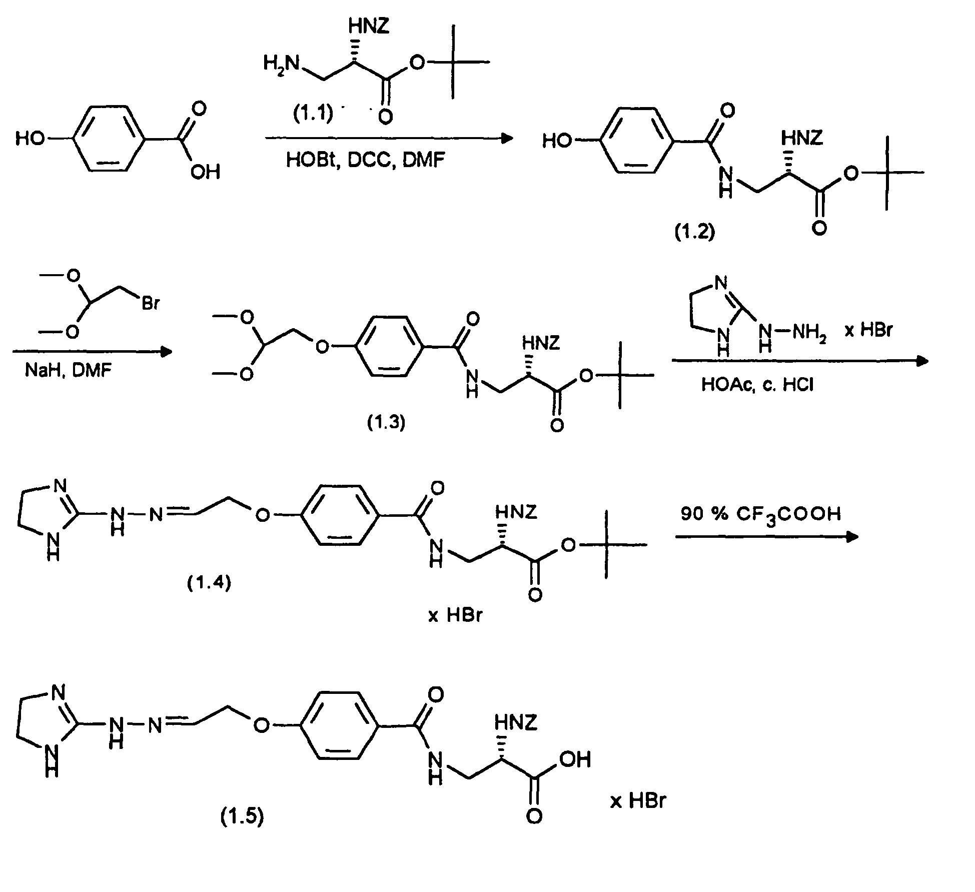

### 1a) (2S)-3-Amino-2-benzyloxycarbonylamino-propionsäure-tert.-butylester (1.1)

10 g (42 mMol) (2S)-3-Amino-2-benzyloxycarbonylamino-propionsäure wurden in einem Gemisch aus 100 ml Dioxan, 100 ml Isobutylen und 8 ml konz. H₂SO₄ 3 Tage bei 20 atm. N₂-Druck im Autoklaven geschüttelt. Überschüssiges Isobutylen wurde abgeblasen und zur verbleibenden Lösung wurden 150 ml Diethylether und 150 ml gesättigte NaHCO₃-Lösung gegeben. Die Phasen wurden getrennt und die wäßrige Phase wurde 2mal mit je 100 ml Diethylether extrahiert. Die vereinigten organischen Phasen wurden mit 2x100 ml H₂O gewaschen und über Na₂SO₄ getrocknet. Nach Entfernen des Lösungsmittels im Vakuum erhielt man (1.1) als blaßgelbes Öl.

### 1b) 4-Hydroxybenzoyl-(2S)-2-benzyloxycarbonylamino-β-alanin-tert.-butylester (1.2)

1,41 g (10,2 mMol) 4-Hydroxybenzoesäure und 3 g (10,2 mmol) (1.1) wurden in 25 ml DMF suspendiert. Man gab 1,38 g (10,2 mMol) 1-Hydroxybenzotriazol (HOBt) und bei 0°C Dicyclohexylcarbodiimid (DCCI) zu. Man rührte 1 h bei 0°C und ließ über Nacht bei Raumtemperatur stehen. Nach Filtration wurde das Lösungsmittel im Vakuum entfernt und der Rückstand mit Heptan/Essigester (1/1) mittels MPLC über Kieselgel chromatographiert.
Man erhielt (1.2) als farblosen Feststoff; Schmelzpunkt 69°C.

### 1c) 4-(2,2-Dimethoxy-ethyloxy)benzoyl-(2S)-2-benzyloxycarbonylamino-β-alanin-tert.-butylester (1.3)

Man gab zu einer Suspension von 176 mg einer 55 %igen Natriumhydridsuspension in Öl (4,07 mMol an Natriumhydrid) in 10 ml abs. DMF 1,8 g (4,34 mMol) (1.2) und rührte, bis die Wasserstoff-Entwicklung beendet ist (ca. 30 min.). Anschließend gab man 620 mg (3,7 mMol) Bromacetaldehyd-dimethylacetal zu und erhitzte 8 h auf 50°C und 2 h auf 70°C. Nach erneuter Zugabe von 18 mg der Natriumhydridsuspension in Öl (0,41 mMol an Natriumhydrid) wurde weitere 4 h auf 70°C erhitzt. Nach Stehen über Nacht wurde das Reaktionsgemisch einrotiert und der Rückstand zwischen H₂O und CH₂Cl₂ verteilt. Die organische Phase wurde abgetrennt, über MgSO₄ getrocknet und das Lösungsmittel i.Vak. entfernt. Der Rückstand wurde mittels MPLC mit Heptan/Essigester über Kieselgel chromatographiert. Man erhielt (1.3) als farblosen Feststoff; Schmelzpunkt 115°C.

### 1d) 4-[2-(N-(Imidazolin-2-yl)hydrazonoethyloxy)]benzoyl-(2S)-2-benzyloxycarbonylamino-β-alanin-tert.-butylester-Hydrobromid (1.4)

150 mg (0,3 mMol) (1.3) und 54 mg (0,3 mMol) 2-Hydrazino-2-imidazolin-Hydrobromid wurden in 3 ml konz. Essigsäure gelöst und mit 1 Tropfen konz. Salzsäure versetzt. Nach 5 h bei Raumtemperatur wurde das Reaktionsgemisch in Diethylether gegossen. Der Niederschlag wurde abzentrifugiert, mit Diethylether verrieben und erneut abzentrifugiert und nach Trocknen im Vakuum direkt zu (1.5) umgesetzt (siehe 1e)).

### 1e) 4-[2-(N-(Imidazolin-2-yl)hydrazonoethyloxy)]benzoyl-(2S)-2-benzyloxycarbonylamino-β-alanin-Hydrobromid (1.5)

Das Rohprodukt (1.4) aus 1d) wurde mit 90 %iger Trifluoressigsäure versetzt. Nach 1 h bei Raumtemperatur wurde die Trifluoressigsäure im Vakuum entfernt und der Rückstand mit H₂O/n-Butanol/HOAc (43/4,3/3,5) kristallisiert. Man erhielt (1.5) als farblosen Feststoff; Schmelzpunkt 219°C (Zersetzung).

## Patentansprüche

1. Verbindung der Formel I,
R¹-Y-A-B-D-E-F-G I,
worin bedeuten:
A -NH-N = CH-;
B (C₁-C₆)-Alkandiyl;
D -O-;
E Phenylen;
F -CO-NH-;
G 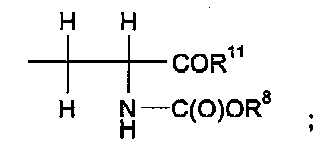
Y eine direkte Bindung;
R¹ H₂N-C(=NH)-, 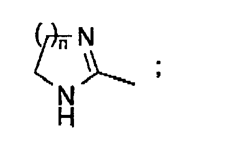
R⁸ H, (C₁-C₆)-Alkyl, (C₅-C₆)-Cycloalkyl, (C₅-C₆)-Cycloalkyl-(C₁-C₂)-alkandiyl, (C₅-C₆)-Aryl, (C₅-C₆)-Aryl-(C₁-C₂)-alkandiyl;
R¹¹ OH, (C₁-C₆)-Alkoxy, Phenoxy, Benzyloxy, (C₁-C₄)-Alkylcarbonyloxy-(C₁-C₄)-alkandiyloxy, NH₂, Mono- oder Di-(C₁-C₆)-alkyl-amino;
n 1 oder 2;
in allen ihren stereoisomeren Formen und Gemische davon in allen Verhältnissen, und ihre physiologisch verträglichen Salze.

2. Verbindung der Formel I gemäß Anspruch 1 und/oder ihre physiologisch verträglichen Salze zur Verwendung als Arzneimittel.

3. Verbindung der Formel I gemäß Anspruch 1 und/oder ihre physiologisch verträglichen Salze als Inhibitoren der Knochenresorption durch Osteoclasten, als Inhibitoren von Tumorwachstum und Tumormetastasierung, als Entzündungshemmer, zur Behandlung oder Prophylaxe von cardiovaskulären Erkrankungen, zur Behandlung oder Prophylaxe von Nephropathien und Retinopathien oder als Vitronectinrezeptor-Antagonisten zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen.

4. Pharmazeutische Zubereitung, enthaltend mindestens eine Verbindung der Formel I gemäß Anspruch 1 und/oder deren physiologisch verträgliche Salze neben pharmazeutisch einwandfreien Träger- und Zusatzstoffen.

## Claims

1. A compound of the formula I,
R¹-Y-A-B-D-E-F-G I,
in which:
A is -NH-N=CH-;
B is (C₁-C₆)-alkanediyl;
D is -O-;
E is phenylene;
F is -CO-NH-;
G is 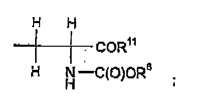
Y is a direct bond;
R¹ is H₂N-C(=NH)-; 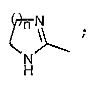
R⁸ is H, (C₁-C₆)-alkyl, (C₅-C₆)-cycloalkyl, (C₅-C₆)-cycloalkyl-(C₁-C₂)-alkanediyl, (C₅-C₆)-aryl, (C₅-C₆)-aryl-(C₁-C₂)-alkanediyl;
R¹¹ is OH, (C₁-C₆)-alkoxy, phenoxy, benzyloxy, (C₁-C₄)-alkylcarbonyloxy-(C₁-C₄)-alkanediyloxy, NH₂, mono-or di-(C₁-C₆)-alkyl-amino;
n is 1 or 2;
in all its stereoisomeric forms and mixtures thereof in all ratios, and its physiologically tolerated salts.

2. A compound of the formula I as claimed in claim 1 and/or its physiologically tolerated salts for use as a pharmaceutical.

3. A compound of the formula I as claimed in claim 1 and/or its physiologically tolerated salts as inhibitors of bone resorption by osteoclasts, as inhibitors of tumor growth and tumor metastasis, as antiinflammatories, for the treatment or prophylaxis of cardiovascular disorders, for the treatment or prophylaxis of nephropathies and retinopathies or as vitronectin receptor antagonists for the treatment and prophylaxis of diseases which are based on the interaction between vitronectin receptors and their ligands in cell-cell or cell-matrix interaction processes.

4. A pharmaceutical preparation comprising at least one compound of the formula I as claimed in claim 1 and/or its physiologically tolerable salts in addition to pharmaceutically innocuous excipients and additives.

## Revendications

1. Composé de formule (I)
R¹-Y-A-B-D-E-F-G I,
où
A signifie -NH-N=CH- ;
B signifie (C₁-C₆)-alcanediyle ;
D -O- ;
E signifie phénylène ;
F signifie -CO-NH- ;
G signifie 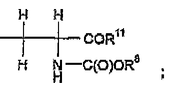
Y signifie une liaison directe ;
R¹ signifie H₂N-C(=NH)-, 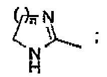
R⁸ signifie H, (C₁-C₆)-alkyle, (C₅-C₆)-cycloalkyle, (C₅-C₆)-cycloalkyl-(C₁-C₂)-alcanediyle, (C₅-C₆)-aryle, (C₅-C₆)-aryl-(C₁-C₂)-alcanediyle ;
R¹¹ signifie OH, (C₁-C₆)-alcoxy, phénoxy, benzyloxy, (C₁-C₄)-alkylcarbonyloxy-(C₁-C₄)-alcanediyloxy, NH₂, mono-(C₁-C₆)-alkylamino ou di-(C₁-C₆)-alkylamino ;
n vaut 1 ou 2 ;
dans toutes ses formes stéréo-isomères et leurs mélanges, dans tous les rapports, et ses sels physiologiquement acceptables.

2. Composés de formule I selon la revendication 1 et/ou ses sels physiologiquement acceptables destinés à une utilisation comme médicament.

3. Composés de formule I selon la revendication 1 et/ou ses sels physiologiquement acceptables comme inhibiteurs de la résorption osseuse par des ostéoclastes, comme inhibiteurs de la croissance et de la formation de métastases de tumeurs, comme anti-inflammatoires, destinés au traitement ou à la prophylaxie de maladies cardiovasculaires, au traitement ou à la prophylaxie de néphropathies et de rétinopathies ou comme antagonistes du récepteur de la vitronectine pour le traitement et la prophylaxie de maladies qui reposent sur l'interaction entre les récepteurs de la vitronectine et leurs ligands dans des processus d'interaction cellule-cellule ou cellule-matrice.

4. Préparation pharmaceutique, contenant au moins un composé de formule I selon la revendication 1 et/ou ses sels physiologiquement acceptables, en plus de supports et d'additifs pharmaceutiquement inoffensifs.
